## Europäisches Patentamt
## European Patent Office
## Office européen des brevets

(11) Publication number: **0 093 546**
A2

# EUROPEAN PATENT APPLICATION

(21) Application number: 83302268.4

(51) Int. Cl.³: **G 01 D 5/20**

(22) Date of filing: 21.04.83

(30) Priority: 21.04.82 GB 8211460

(43) Date of publication of application:
09.11.83 Bulletin 83/45

(84) Designated Contracting States:
AT BE CH DE FR GB IT LI LU NL SE

(71) Applicant: University of Strathclyde
Royal College 204 George Street
Glasgow G1 1XW(GB)

(72) Inventor: Jackson, John
21 Broompark Drive Lesmahagow
Glasgow Scotland(GB)

(72) Inventor: Singh, Amreek
8 Birch Knowe Bishopbriggs
Glasgow G64 1TS Scotland(GB)

(74) Representative: MacDougall, Donald Carmichael et al,
Messrs. Cruikshank & Fairweather 19 Royal Exchange
Square
Glasgow G1 3AE, Scotland(GB)

(54) Displacement sensitive transducers.

(57) A displacement sensitive transducer (1) comprises a body (2) made of elastomeric material such as silicone rubber in which there is provided a means (4, 10) for supporting a magnetic field within the body (2). A detector arrangement (6) incorporating a magnetic field sensor (7) is provided for detecting variations in the magnetic field of said supporting means (4,10).

Croydon Printing Company Ltd.

## DISPLACEMENT SENSITIVE TRANSDUCERS

This invention relates to displacement sensitive transducers.

Known forms of displacement sensitive transducers incorporate an electrically conductive element through which an electrical current is passed and variation in that current arising from variation in the characteristics of the conductive element caused by displacement of an article being monitored gives rise to a measure of these displacements.

It is an object of the present invention to provide a displacement sensitive transducer which does not rely upon variation in the characteristics of an electrically conductive element.

According to the present invention there is provided a transducer sensitive to displacements of an article being monitored said transducer comprising a body of elastomeric material, means in said body for supporting a magnetic field in said body and detector means comprising a magnetic field sensor for detecting variations in the magnetic field supported by said supporting means.

The elastomeric body is preferably a silicone rubber such as EP411 or C2005. In one form the supporting means in the body comprises a plurality of magnetic particles distributed through the body preferably in a homogeneous fashion. In this arrangement the detector means may be separate from the body. The magnetic particles may be iron particles or iron oxide particles, preferably of the ferric type ($Fe_2O_3$ ). It is preferred that the particles are present in an amount within the range 50-100% parts by weight of the particles to parts by weight of the elastomeric material since this range retains good elastomeric properties and provides good magnetic field sensitivity.

In another form the magnetic field supporting means comprises an energised electrical coil mounted in the body of elastomeric material and in this arrangement the detector means may also be mounted in or on the elastomeric body.

Transducers in accordance with the present invention have a variety of uses, a particular one of which provides the elastomeric body secured to an article the displacements of which are to be monitored with the detector means separate from the body and therefore not in contact with the article.

Embodiments of the present invention will now be described by way of example with reference to the accompanying drawings in which:

Fig. 1 is a schematic illustration of a first embodiment of transducer in accordance with the present invention;

Fig. 2 illustrates a second embodiment of transducer in accordance with the present invention;

Fig. 3 illustrates a third embodiment of transducer in accordance with the present invention; and

Fig. 4 illustrates a displacement monitoring system incorporating a transducer according to the present invention.

In the first embodiment a transducer 1 comprises a body 2 of elastomeric material incorporating particles 4 substantially homogeneously dispersed within the body 2, the particles being capable of supporting a magnetic field within the body 2. For attachment of the body 2 to an article 5 the displacements of which are to be measured one surface of the body 2 is provided with a coating 3 of adhesive material.

The body 2 is preferably made of silicone rubber such as EP411 and the particles 4 are preferably iron particles or iron oxide particles (of the ferric type - $Fe_2O_3$). A detector device 6 incorporating a magnetic field sensor 7 is provided in proximity with the body 2 and the arrangement is such that displacements of the article 5 result in modification of the geometric arrangement of the body 2 and of the magnetic particles 4 which results in variation of the magnetic field in the proximity of the sensor 7 which consequentially provides a variable output electrical signal which is a measure of these displacements.

We have tested transducers 1 incorporating various quantities of particles 4 in order to ascertain the mechanical and electrical properties of the transducers and the results are tabulated in Table I.

TABLE I

| Particle Quantity (% Fe/ polymer) | Tensile Strain (% extension) | Tensile Stress (kg/cm$^2$) | Signal Strength (µV) |
|---|---|---|---|
| 0 | 155.4% | 0.276 | 0 |
| 20 | 123.4 | 0.159 | 12.4 |
| 40 | 139.4 | 0.152 | 20.6 |
| 60 | 99.3 | 0.136 | 60.6 |
| 80 | 118.5 | 0.168 | 140.0 |
| 100 | 109.8 | 0.109 | 41.2 |
| 150 | 94.5 | 0.105 | 66.6 |

It will be observed that as the quantity of particles 4 increases from zero the percentage extension which the body 2 is capable of accommodating prior to rupture decreases as does the tensile stress which requires to be applied to the body 2 to obtain that rupture. However the signal strength as measured by the amplitude of the electrical signal output by the sensor 7 arising from a predetermined deflection at one end of a strip-like transducer 1 passes through a maximum level at a particle loading of about 80% parts by weight particle to parts by weight elastomeric material.  In fact in order to provide a compromise between good mechanical properties and good electrical properties we prefer to have particle loadings in the range 50-100% (parts by weight particles to parts by weight elastomeric material). The particles 4 for these tests were in the form of iron particles having a particle size 200 microns or less and the elastomeric material was a silicone rubber, namely C2501 as manufactured by J-Sil Ltd. This is a room temperature vulcanising silicone rubber and we have found that during the finite curing time the magnetic particles 4 tend to migrate from an homogeneous dispersion towards a sedimentary layer for high particle loadings. We believe that this problem can be overcome even at high particle loadings by using a high temperature vulcanising silicone rubber in which the curing time is extremely short.

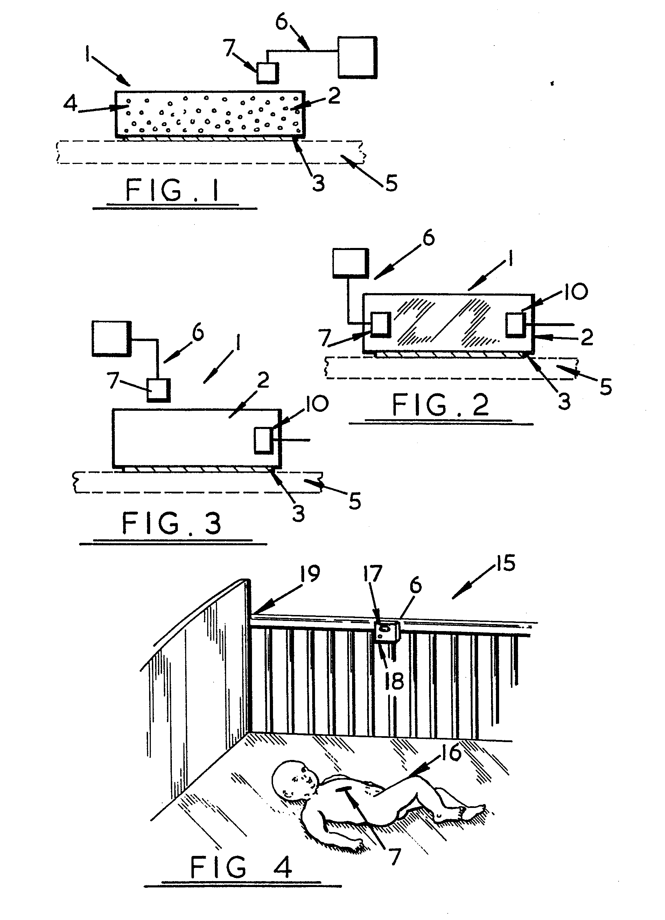

In the embodiment of Fig. 2 the body 2 of elastomeric material does not incorporate magnetic particles 4 but instead the means of supporting a magnetic field within the body 2 is formed by an energised electrical coil 10. In this embodiment the sensor 7 is also implanted into the body 2 and the arrangement is such that the magnetic field established by coil 10 within the body 2 is sensed by sensor 7 so that variations in that magnetic field arising as a result of variations in the geometrical shape of the body 2 and the inherent magnetic field attenuation properties of the elastomeric material used to form the body 2 result in variations in the electrical signal output by the sensor 7.

In the embodiment of Fig. 3 the arrangement is similar to that of Fig. 2 except that the sensor 7 is external to the body 2.

In each of the embodiments the sensor 7 may be that marketed by Radio Spares under their stock number 304-166 and, the same component may be used to form the electrical coil 10.

As is illustrated in Fig. 4 the transducer 1 may be used as part of a system 15 for monitoring the respiration of an infantile patient 16 so that the transducer 1 provides an output signal consequential to the regular breathing of the patient 16 and the detector means 6 is effective to operate an alarm device 17 in the event of an absence of output signal. An alarm reset button 18 is provided.

In the system 15 the transducer 1 could alternatively be mounted in the support structure or mattress of the cot 19 and where the transducer 1 incorporates the sensor 7 an ultrasonic or infra red coupling may be provided with the detector means 6 mounted on the cot wall so that the transducer 1 is free from electrical wires. Advantages of the system 15 are that the overall cost is low, no electrical power is near or in contact with the patient 16 and no electrical wires are attached to the patient 16.

## CLAIMS

1. A transducer sensitive to displacements of an article being monitored said transducer being characterised by a body (2) of elastomeric material, means (4,10) in said body (2) for supporting a magnetic field in said body (2) and detector means (6) comprising a magnetic field sensor (7) for detecting variations in the magnetic field supported by said supporting means (4,10).

2. A transducer as claimed in claim 1, characterised in that said magnetic field supporting means (4,10) comprises a plurality of magnetic particles (4) distributed through the body (2) and said detector means (6) is separate from the body (2).

3. A transducer as claimed in claim 2, characterised in that said magnetic particles (4) are particles of iron.

4. A transducer as claimed in any preceding claim, characterised in that said body (2) is composed of silicone rubber.

5. A transducer as claimed in claim 1, characterised in that said body is composed of silicone rubber and said particles (4) are iron particles which are present in an amount within the range 50-100% parts by weight particles to parts by weight silicone rubber.

6. A transducer as claimed in claim 1, characterised in that said magnetic field supporting means (4,10) comprises an energised electrical coil (10).

7. A transducer as claimed in claim 6, characterised in that said magnetic field sensor (7) is mounted on said body (2).

FIG. 1

FIG. 2

FIG. 3

FIG 4

0093546